# EUROPÄISCHE PATENTSCHRIFT

(11) **EP 3 897 441 B1**
(45) Veröffentlichungstag und Bekanntmachung des Hinweises auf die Patenterteilung: **03.05.2023**
(21) Anmeldenummer: 19832385.9
(22) Anmeldetag: 20.12.2019
(51) Int. Cl.: A61B 42/10, A61B 42/20, G01L 5/00, A61B 34/00, A61B 90/00, G01L 1/24

(54) **FINGERSEGMENT FÜR EINEN HANDSCHUH**
FINGER SEGMENT FOR A GLOVE
SEGMENT DE DOIGT POUR UN GANT

(30) Priorität: 20.12.2018 DE 102018222633
(43) Veröffentlichungstag der Anmeldung: 27.10.2021
(73) Patentinhaber: Fraunhofer-Gesellschaft zur Förderung der angewandten Forschung e.V., 80686 München (DE); Winde, Günther, 32545 Bad Oeynhausen (DE)
(72) Erfinder: WAGNER, Jürgen, 14476 Golm (DE); RABE, Christian, 14476 Golm (DE); WINDE, Günther, 32545 Bad Oeynhausen (DE)
(74) Vertreter: Pfenning, Meinig & Partner mbB
(86) Internationale Anmeldenummer: PCT/EP2019/086699
(87) Internationale Veröffentlichungsnummer: WO 2020/127986

(56) Entgegenhaltungen:
- WO-A1-2016/070078
- WO-A1-2018/138095
- DE-A1-102009 035 363
- US-A1- 2013 225 939
- ARNO SEEBOTH ET AL: "Piezochromic Polymer Materials Displaying Pressure Changes in Bar-Ranges", AMERICAN JOURNAL OF MATERIALS SCIENCE, vol. 1, no. 2, 31 August 2012 (2012-08-31) , pages 139-142, XP055200213, ISSN: 2162-9382, DOI: 10.5923/j.materials.20110102.23

## Beschreibung

Die vorliegende Erfindung betrifft ein Fingersegment für einen Handschuh für medizinische und diagnostische Zwecke, welches ein Sensormaterial enthält, das beim Einwirken einer mechanischen Kraft eine charakteristische Änderung erfährt, die für den Nutzer ohne technische Hilfsmittel erkennbar ist. Weiterhin betrifft die vorliegende Erfindung einen Handschuh, der dieses Fingersegment aufweist und Verwendungen des Fingersegments oder des Handschuhs.

Medizinische Handschuhe bzw. Fingersegmente für diese Handschuhe kommen in nahezu allen medizinischen Versorgungssituationen zum Einsatz und dienen der Vermeidung eines direkten Kontakts zwischen Patient und Anwender. Sie bestehen aus einem Polymermaterial und sind für den einmaligen Gebrauch vorgesehen. Diese Handschuhe werden in sterilen und nichtsterilen Varianten hergestellt und gehören zur persönlichen Schutzausrüstung von Medizinern und Pflegepersonal. Durch eine anschmiegsame Passform wird ein hoher Tragekomfort aber auch eine unbeeinträchtigte haptische Wahrnehmung gewährleistet. Diagnostische und therapeutische Aufgaben können mit diesen Handschuhen leicht durchgeführt werden.

Neben den Grundanforderungen an einen medizinischen Handschuh ist für besondere medizinische Aufgaben die Integration zusätzlicher Funktionen vorteilhaft. Insbesondere bedeutsam ist die Integration von zusätzlichen Funktionen in einen medizinischen Handschuh für die Herstellung einer chirurgischen Naht.

Zum Verschließen und Fixieren einer Operationswunde erstellt der Operateur eine chirurgische Naht. Dabei werden zunächst die Wundränder durch das Nahtmaterial miteinander verbunden. Durch einen Zug am Nahtmaterial wird ein direkter Kontakt der Ränder hergestellt, ohne dass angrenzendes Gewebe geschädigt wird. Anschließend wird die Naht mit einem chirurgischen Knoten fixiert. Der Vorgang erfordert einen präzisen Krafteinsatz durch den Operateur. Hierbei wird das Nahmaterial über einen Finger geleitet, der die Zugkraft auf Naht und Knoten überträgt. Dabei übt der Faden einen Druck auf seine Auflagefläche aus. Dieser Druck ist somit ein Maß für die geleistete Zugkraft. Die erforderlichen Zugkräfte und resultierenden Auflagedrücke variieren mit den zu vernähenden Gewebearten. Der Operateur ist bei der Dosierung der Kraft rein auf Erfahrungswerte angewiesen. Insbesondere Anfängern fällt es sehr schwer die benötigte Kraft abzuschätzen. Derzeit gibt es keine technischen Hilfsmittel, die für die Unterstützung in geeigneter Weise eingesetzt werden können.

Die vorliegende Erfindung zielt auf Anwendungsszenarien, insbesondere im Medizinbereich, ab, bei denen es erforderlich ist, mit der Hand oder mit den Fingern ausgeübte Kräfte genau zu dosieren. Anwendungsfelder liegen neben der Chirurgie beispielsweise in der Aus- und Fortbildung von Medizinern und therapeutischem Personal.

Medizinische Handschuhe ohne zusätzliche Funktionen sind im Stand der Technik beschrieben.

WO 2018/138095 A1 offenbart einen doppellagigen Handschuh der einen Defekt im Material visuell anzeigen kann (gemäß der Präambel aus Anspruch 1).

DE 10 2005 009 826 A1 betrifft einen Handschuh für medizinische Zwecke, insbesondere einen Schutzhandschuh zur Verwendung bei medizinisch diagnostischen Untersuchungen an/in Körperöffnungen eines Patienten, bestehend aus einer dünnen, die Hand des Untersuchenden eng umschließenden elastischen Schicht, insbesondere Gummi-, Kunststoff- oder Latexschicht, mit daran angeformten Aufnahmebereichen für die Finger, wobei zumindest die Schichtdicke des Aufnahmebereiches für den Mittelfinger gegenüber dem Handflächenaufnahmebereich zumindest bereichsweise entweder verstärkt oder doppelwandig ausgebildet ist.

WO 2008/135213 A1 beschreibt einen wegwerfbaren mehrlagigen medizinischen Handschuh aus polymerem Material mit einem Fingerbereich, einem Handflächenbereich und einem Stulpenbereich, mit einem vorderen Abschnitt mit einem geschlossenen Ende und einem hinteren Abschnitt mit offenem Ende, wobei der vordere Abschnitt den Fingerbereich vollständig und den Handflächenbereich zumindest bereichsweise enthält und wobei eine erste innere polymere Lage den vorderen Abschnitt und den hinteren Abschnitt umfasst und wobei die innere Lage in dem vorderen Abschnitt mit einer zweiten äußeren polymeren Lage zur Bildung eines Verstärkungsbereiches beschichtet ist und wobei der Verstärkungsbereich den vorderen Abschnitt bildet und begrenzt.

Die Integration zusätzlicher Funktionen in ein Handschuhsystem ist ebenso bekannt.

WO 2016/070078 A1 offenbart Sensorsysteme, die dazu bestimmt sind, in Handschuhe für die menschliche Hand integriert zu werden. Eine Anordnung von Sensoren erfasst Kräfte, die mit der Aktion einer Hand in dem Handschuh verbunden sind, und zugehörige Schaltungen erzeugen entsprechende Steuerinformationen, die zur Steuerung einer großen Vielzahl von Prozessen und Vorrichtungen verwendet werden können.

DE 10 2010 019 573 A1 beschreibt einen Schutzhandschuh, der aus einem säure-/base-resistenten Stoff besteht, auf dessen Oberfläche sich eine Beschichtung befindet, die pH-Indikatoren enthält, die sich bei für die Haut gefährlichen pH-Werten verfärben und dass die Beschichtung bestehend aus einer Lösung aus 3-Methacryloxypropyltrimethoxysilan, SiO₂-Nanopartikeln, Phenyltrimethoxysilan, Isopropanol, Methacrylsäure, einer radikalischen Photostarter-Lösung und sulfonierten Tetrafluorethylen-Polymeren, Phenolphthalein und Bromkresolgrün-Natrium-Salz durch Sprühen oder Fluten auf den Handschuh aufgetragen wird und die Aushärtung mittels UV-Lampe erfolgt.

Eine Funktionalisierung von Handschuhen bzw. Fingersegmenten, die es dem Nutzer erlaubt, die mit der Hand bzw. den Fingern ausgeübte mechanische Kraft zu dosieren, ist bislang nicht beschrieben worden.

Ausgehend davon bestand die Aufgabe der vorliegenden Erfindung darin, ein Fingersegment für einen Handschuh bereitzustellen, welches es dem Nutzer ermöglicht die ausgeübte Kraft möglichst exakt zu dosieren, ohne dass er hierzu technische Hilfsmittel benötigt.

Diese Aufgabe wird durch das Fingersegment für einen Handschuh für diagnostische und medizinische Zwecke mit den Merkmalen von Anspruch 1 gelöst. Dieses Fingersegment enthält ein den Finger des Nutzers umschließendes, elastisches Material und ein vom elastischen Material eingeschlossenes Sensormaterial, wobei das Sensormaterial geeignet ist, eine einwirkende mechanische Kraft anzuzeigen und wobei das Sensormaterial eine für die einwirkende mechanische Kraft charakteristische Änderung erfährt, die für den Nutzer ohne technische Hilfsmittel erkennbar ist. Das Fingersegment ist dadurch gekennzeichnet, dass das Sensormaterial dazu geeignet ist, mechanische Kräfte im Bereich von 1 bis 25 N, die auf das Sensormaterial in unterschiedlichen Auftreffwinkeln wirken, zu visualisieren.

Vorteilhafte Eigenschaften des erfindungsgemäßen Fingersegments werden in den abhängigen Ansprüchen 2 bis 13 angegeben.

Anspruch 14 betrifft einen Handschuh der mindestens ein Fingersegment gemäß einem der Ansprüche 1 bis 13 aufweist.

Weiterhin betrifft Anspruch 15 Verwendungen des Fingersegments nach einem der Ansprüche 1 bis 13 oder des Handschuhs gemäß Anspruch 14.

Ein "Fingersegment" eines Handschuhs meint im Sinne der vorliegenden Erfindung den Fingerbereich eines Handschuhs, der geeignet ist den Finger des Nutzers vollständig zu umschließen. Der Begriff "umschließen" meint dabei, ein vollständiges und enganliegendes umhüllen des Fingers, wobei der Finger abgebildet wird, ohne dass es zu einer Beeinträchtigung des Trägers kommt.

### Fingersegment

Im Folgenden werden bevorzugte Ausführungsformen des erfindungsgemä-ßen Fingersegments angegeben. Die vorliegende Erfindung eines Fingersegments wird im Hauptanspruch 1 definiert. Hauptanspruch 16 definiert die Verwendung dieses Fingersegments. Die weiteren Ausführungsformen werden in den Nebenansprüchen definiert.

Gemäß einer bevorzugten Ausführungsform des erfindungsgemäßen Fingersegments ist das elastische Material ausgewählt aus der Gruppe bestehend aus transluzenten Latexmaterialien, insbesondere Nitrilkautschuken, Gummimaterialien, Kunststoffmaterialien, insbesondere Polyvinylchlorid, Polyethylen, Neopren, Styren-Butadien-Polymeren und Mischungen hiervon.

Eine andere erfindungsgemäße bevorzugte Ausführungsform sieht vor, dass das Fingersegment zumindest teilweise und bevorzugt vollständig einen mehrschichtigen Aufbau aufweist, wobei ein dreischichtiger Aufbau besonders bevorzugt ist und insbesondere bevorzugt eine innere Schicht, die das Sensormaterial enthält von zwei äußeren Schichten, die das elastische Material enthalten, eingeschlossen ist. Dabei beträgt die Schichtdicke der Schicht mit dem Sensormaterial bevorzugt von 50 bis 100 µm und besonders bevorzugt von 50 bis 60 µm und die Gesamtdicke des Mehrschichtaufbaus bevorzugt von 150 bis 1000 µm und besonders bevorzugt von 150 bis 500 µm.

Eine andere bevorzugte Ausführungsform des erfindungsgemäßen Fingersegments sieht vor, dass das elastische Material den Finger des Nutzers vollständig umschließt.

Gemäß einer weiteren bevorzugten Ausführungsform der vorliegenden Erfindung besteht das Fingersegment aus dem elastischen Material und dem Sensormaterial.

Nach einer anderen bevorzugten Ausführungsform vorliegender Erfindung wird das Sensormaterial durch ein Verfahren ausgewählt aus der Gruppe bestehend aus Rakeln, Tauchen, Sprühen, Drucken und Kleben auf dem elastischen Material als Schicht auf einer Schicht des elastischen Materials fixiert, wobei es bei Rakeln, Tauchen, Sprühen und Drucken bevorzugt ist, dass das Sensormaterial vollständig vom elastischen Material umhüllt wird.

Eine andere bevorzugte Ausführungsform der vorliegenden Erfindung sieht vor, dass das Sensormaterial in allen Bereichen des Fingerelements eine identische Empfindlichkeit aufweist.

Gemäß einer weiteren bevorzugten Ausführungsform vorliegender Erfindung ist das Sensormaterial in verschiedenen Bereichen des Fingerelements angebracht und weist jeweils unterschiedliche Empfindlichkeiten auf, wobei die Differenz in der Empfindlichkeit mindestens 5 N, bevorzugt mindestens 8 N beträgt. Dabei ist es bevorzugt, dass die Einstellung der Empfindlichkeit des Sensormaterials während des Herstellprozesses erfolgt, besonders bevorzugt erfolgt die Einstellung der Empfindlichkeit durch Materialwahl des Sensormaterials und/oder durch die Einstellung der Schichtdicke.

Nach einer weiteren bevorzugten Ausführungsform der vorliegenden Erfindung ist die charakteristische Änderung des Sensormaterials durch die Einwirkung von mechanischer Kraft eine Farbänderung im sichtbaren Bereich des elektromagnetischen Spektrums und kann reversibel oder irreversibel erfolgen, wobei ein reversibler Farbwechsel bevorzugt ist. Dabei ist es bevorzugt, dass die Farbänderung im sichtbaren Bereich des elektromagnetischen Spektrums nur durch einen zusätzlichen Stimulus ausgelöst wird, bevorzugt ist dieser zusätzliche Stimulus die Einwirkung von Licht einer Wellenlänger von < 400 nm, bevorzugt < 360 nm, insbesondere im Bereich von 280 bis 400 nm und besonders bevorzugt im Bereich von 280 bis 360 nm.

Nach einer anderen bevorzugten Ausführungsform der vorliegenden Erfindung ist das Sensormaterial ein Material, welches herstellbar aus einem Gemisch aus einem Flüssigkristall, einer optisch aktiven Substanz, einem Photoinitiator einem Netzwerkmonomer und einem Vernetzer ist. Dabei ist der Flüssigkristall bevorzugt ausgewählt aus der Gruppe bestehend aus Benzoesäurecholesterylester, Cholesterin, Tolan, Alkansäuren, Stilben, Azobenzolen, N-(p-Ethoxy-benzyliden)-p-n-butylanilin, N-(p-Methoxy-benzyliden)-p-n-butylanilin, Derivaten des 4-n-Alkylbenzoesäure-(4-alkyl-phenylesters), 4-Phenylzimtsäure, p-Terphenyl, 1,2-Bis-benzoylethen, Flüssigkristallmischungen von Alkylcyanobiphenyl- und Alkoxycyanobiphenyl-derivaten und Mischungen hiervon. Die optische aktive Substanz ist bevorzugt ein 4-(4-Hexyloxybenzoyloxy)benzoat oder ein Cholesterylderivate. Die netzwerkbildenden Monomere sind bevorzugt Verbindungen ausgewählt aus der Gruppe bestehend aus Benzylmethacrylat, 2-Ethylhexylacrylat, 2-Methoxyethylacrylat, Octadecylacrylat, Reaktivmesogenen und Mischungen hiervon.

Der Vernetzer ist bevorzugt ausgewählt aus der Gruppe bestehend aus 1,4-Butandiol-diacrylat, Polyethylenglycol-diacrylat, 1,10-Decandioldiacrylat und Mischungen hiervon. Der Photoinitator ist bevorzugt ausgewählt aus der Gruppe bestehend aus Benzilketalen, α-Dialkoxyacetophenonen, α-Hydroxyalkylphenolen, α-Aminoalkylphenonen, Acylphosphinoxiden, Benzophenonen, Photosäuren und Mischungen hiervon.

Bevorzugte Photoinitatoren sind Irgacure 250 (BASF), Genocure LTM (Rahn) und Irgacure 2959 (Sigma Aldrich).

Die Herstellung geeigneter Photomaterialien wurde in der Literatur beschrieben. So beispielsweise von Stumpel, J.E., Broer, D.J., Schenning, A.P.H.J. im Artikel "Stimuli-responsive photonic polymer coatings" erschienen in Chem. Commun., 2014, 50, Seite 15839 bis 15848, von Seeboth, A., Loetzsch, D. Ruhmann, R. im Artikel "Piezochromic Polymer Materials Displaying Pressure Changes in Bar-Ranges" erschienen in American Journal of Materials Science 2011, 1 (2), Seite 139 bis 142 oder in DE 10 2009 035 363 A1.

Dabei ist es besonders bevorzugt, dass ein Gemisch verwendet wird, welches den Vernetzer mit bis zu 20 Gew.-%; das netzwerkbildende Monomer und den Flüssigkristall mit bis zu 90 Gew.-%, den Photoinitiator mit bis zu 2 Gew.-% und die optisch aktive Substanz mit bis zu 30 Gew.-% enthält.

Gemäß einer weiteren bevorzugten Ausführung vorliegender Erfindung ist die charakteristische Änderung die das Sensormaterial erfährt für Maschinen lesbar und/oder auswertbar. Eine besonders bevorzugte charakteristische Änderung ist hierbei ein Farbwechsel.

Erfindungsgemäß kann das Sensormaterial mechanische Kräfte im Bereich von 1 bis 25 N und bevorzugt von 5 bis 10 N visualisieren, die auf das Sensormaterial oder die das Sensormaterial enthaltende Schicht in unterschiedlichen Auftreffwinkeln wirken. Die dabei senkrecht auf die Sensorschicht wirkenden Kräfte leiten sich aus der Summe aller wirkenden Kräfte ab. Für den Einsatz an parenchymatösem Gewebe, insbesondere an Leber- und Darmgewebe ist eine Visualisierung von Kräften im Bereich von 3 bis 10 N bevorzugt und besonders bevorzugt 4 N. Für Muskelgewebe ist eine Visualisierung von Kräften im Bereich von 10 bis 21 N bevorzugt und besonders bevorzugt von 10 bis 12 N.

Nach einer anderen bevorzugten Ausführungsform der vorliegenden Erfindung kann das Sensormaterial Drücke im Bereich von 0,1 bis 5 bar und bevorzugt von 0,5 bis 2,5 bar visualisieren, die senkrecht zur Flächennormalen des Sensormaterials oder der das Sensormaterial enthaltenden Schicht wirken.

Eine weitere bevorzugte erfindungsgemäße Ausführungsform sieht vor, dass das Fingersegment geeignet ist
a) anzuzeigen ob eine mechanische Krafteinwirkung erfolgte; und
b) diese Krafteinwirkung zu quantifizieren.

Nach einer anderen bevorzugten Ausführungsform der vorliegenden Erfindung wird die Intensität der mechanischen Krafteinwirkung durch unterschiedliche Farben visualisiert, wobei es besonders bevorzugt ist, dass eine zunehmende Einwirkung der mechanischen Kraft durch eine Blauverschiebung angezeigt wird.

Eine weitere erfindungsgemäße Ausführungsform sieht vor, dass die charakteristische Änderung des Sensormaterials auch in den an die Bereiche in denen mechanische Krafteinwirkung erfolgte direkt benachbarten Bereichen angezeigt wird, besonders bevorzugt durch einen Farbumschlag.

Nach einer anderen erfindungsgemäßen Ausführungsform erfüllt das Fingersegment die Erfordernisse der Europäischen Richtlinie 93/42/EWG und/oder der Europäischen Norm EN 455-1 bis 4.

Gemäß einer weiteren Ausführungsform der vorliegenden Erfindung ist das Fingersegment als Fingerling ausgebildet.

### Handschuh

Die vorliegende Erfindung betrifft auch einen Handschuh der zumindest ein erfindungsgemäßes Fingersegment umfasst.

Dabei sind bevorzugtermaßen auch die übrigen Teile des Handschuhs steril und erfüllen besonders bevorzugt die Erfordernisse der Europäischen Richtlinie 93/42/EWG und/oder der Europäischen Norm EN 455-1 bis 4.

### Verwendung

Die vorliegende Erfindung betrifft auch die Verwendung des erfindungsgemä-ßen Fingersegments oder des erfindungsgemäßen Handschuhs in der Ausbildung von Medizinern, im Bereich der Chirurgie, insbesondere der Thorax-, Gefäß-, Viszeral- und Neurochirurgie, Orthopädie, Frauenheilkunde, Zahnheilkunde, Physiotherapie, Feinmechanik und bei der CD-Herstellung.

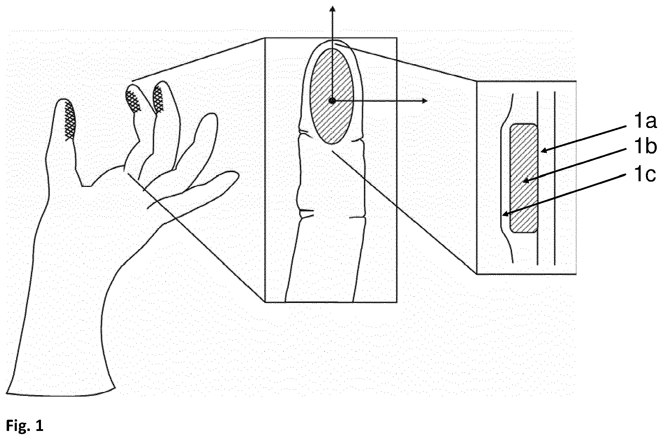

Im linken Teil von Figur 1 wird ein medizinischer Handschuh abgebildet, der die erfindungsgemäßen Fingersegmente aufweist. Der schraffierte Bereich enthält dabei das Sensormaterial. Im mittleren Teil von Figur 1 wird eine Vergrößerung eines erfindungsgemäßen Fingersegments gezeigt, wobei der Blickwinkel entlang der Flächennormalen ist. Der rechte Teil der Figur 1 bildet einen Mehrschichtaufbau des erfindungsgemäßen Fingersegments im Querschnitt ab. Dieser Mehrschichtaufbau weist eine Grundschicht (1a), eine piezochrome Sensorschicht (1b) und eine Deckschicht (1c) auf.

Anhand der nachfolgenden allgemeinen Herstellvorschrift soll der erfindungsgemäße Gegenstand näher erläutert werden, ohne diesen auf die hier gezeigten spezifischen Ausführungsformen einschränken zu wollen.

### Allgemeine Herstellungsvorschrift

Für die Fertigung des erfindungsgemäßen Fingersegments eines Handschuhs kann ein anatomisch geformtes Formwerkzeug mit zumindest einem Fingerbereich genutzt werden. Für die Ausfertigung als Handschuh kann dieses Formwerkzeug um einen Handflächenbereich und einen Stulpenbereich ergänzt werden. Zur Erzeugung unterschiedlicher Passgrößen ist es möglich das Formwerkzeug in seiner Größe variabel auszuführen. Das Formwerkzeug kann aus einem für Tauchbeschichtungen geeigneten Material gefertigt werden und besteht bevorzugt aus technischem Porzellan. Die Form kann bis in den bevorzugten Bereich des Formwerkzeugs in die jeweiligen Tauchbäder abgesenkt werden und nach der jeweiligen Verweildauer wieder herausgehoben werden.

Als Koagulationshilfsmittel zur Abscheidung von Polymeren aus einer entsprechenden Dispersion können Salzlösungen eingesetzt werden. Die Schichtdicke des Überzuges kann einerseits durch die Konzentration der Salzlösung, in die das Formwerkzeug zunächst getaucht wird, gesteuert werden, aber auch durch die Verweildauer in der Polymerdispersion und deren Konzentration im sich anschließenden Tauchprozessschritt. Der polymere Vorläufer in der Dispersion liegt beispielsweise in Form von natürlichem Kautschuk oder von synthetischen Polyisoprenen und Acrylonitril-Butadienmaterialien und deren Kombinationen vor. Nach Herausheben aus der Polymerdispersion hat sich ein Polymerfilm definierter Schichtdicke auf dem Formwerkzeug abgeschieden.

Das Sensormaterial kann beispielsweise aus einem Gemisch aus Flüssigkristall, optisch aktiver Substanz, Netzwerkmonomer, Vernetzer und Photoinitiator hergestellt werden. Dieses Gemisch bildet eigenständig eine linksgängig oder rechtsgängig helikal geordnete Struktur aus, die durch thermische Behandlung zusätzlich gesteuert werden kann.

Die nachfolgende Aufstellung ist nur als Auswahl an Substanzen zu verstehen. Dabei ist für die Sensorfunktion maßgeblich das molekulare Verhältnis der Komponenten zueinander von Bedeutung. Ein bevorzugtes Gemisch enthält den Vernetzer mit bis zu 20 Gew.-%; Netzwerkmonomer und Flüssigkristall mit bis zu 90 Gew.-%, den Photoinitiator mit bis zu 2 Gew.-% und die optisch aktive Substanz mit bis zu 30 Gew.-%.

Bevorzugte Flüssigkristalle sind ausgewählt aus der Gruppe bestehend aus Benzoesäurecholesterylester, Cholesterin, Tolan, Alkansäuren, Stilben, Azobenzolen, N-(p-Ethoxy-benzyliden)-p-n-butylanilin, N-(p-Methoxy-benzyliden)-p-n-butylanilin, Derivaten des 4-n-Alkylbenzoesäure-(4-alkyl-phenylesters), 4-Phenylzimtsäure, p-Terphenyl, 1,2-Bis-benzoylethen, Flüssigkristallmischungen von Alkyl-cyanobiphenyl- und Alkoxycyanobiphenylderivaten und Mischungen hiervon.

Die optische Aktivität kann beispielsweise durch den Zusatz von 4-(4-Hexyloxybenzoyloxy)benzoaten oder Cholesterylderivaten erzielt werden.

Als netzwerkbildende Monomere werden bevorzugt Verbindungen ausgewählt aus der Gruppe bestehend aus Benzylmethacrylat, 2-Ethylhexylacrylat, 2-Methoxyethylacrylat, Octadecylacrylat, Reaktivmesogenen und Mischungen hiervon eingesetzt.

Als Vernetzer kommen bevorzugt Verbindungen ausgewählt aus der Gruppe bestehend aus 1,4-Butandiol-diacrylat, Polyethylenglycol-diacrylat, 1,10-Decandioldiacrylat und Mischungen hiervon zum Einsatz.

Als Polymerisationsinitiatoren sind Substanzen mit einem optischen gesteuerten Radikalbildungsmechanismus gegenüber den Substanzen mit einem thermischen Radikalbildungsverhalten bevorzugt. Diese Substanzen aus dem Bereich der Photoinitiatoren umfassen beispielsweise Benzil Ketale, α-Dialkoxyacetophenone, α-Hydroxy-alkylphenone, α-Amino-alkylphenone, Acylphosphinoxide oder Benzophenone, Irgacure 250 (BASF), Genocure LTM (Rahn) oder Irgacure 2959 (SIGMA ALDRICH).

Eine bevorzugte chirale Flüssigkristallphase kann durch das Mischen der Komponenten p-Ethoxy(benzyliden)-p-n-butylanilin und N-(4-Methoxybenzyliden)-4-butylanilin im Verhältnis 3 : 1 erhalten werden, wenn die Gesamtmischung die optisch aktive Substanz (4-[[4-(Hexyloxy)benzoyl]oxy]benzoesäure-2-octylester) mit 30 Gewichtsprozent enthält und für mehrere Minuten auf über 50 °C erhitzt und anschließend abgekühlt wird.

Eine bevorzugte netzwerkbildende Phase kann durch Mischen von Methylmethacrylat mit einer 30 Gew.-%igen Mischung von 1,4-Butandiol-diacrylat und 1,10-Decandioldiacrylat im Verhältnis 1 : 2 erzeugt werden. Dieser Mischung werden 0,5 Gew.-% des Photoinitiators Genocure LTM beigemischt und anschließend wird die Mischung unter Lichtausschluss weiterverwendet. Die flüssigkristalline Phase und die netzwerkbildende Phase werden daraufhin vereinigt und für das Beschichtungsprocessing bei über 50 °C homogenisiert.

Im dafür vorgesehen Bereich des Überzugs wird zunächst eine Orientierungsschicht hergestellt, die dem Sensormaterial zugewandt ist. Die Herstellung dieser Schicht kann im einfachsten Fall durch mechanische Strukturierung mittels Reiben über einen Polyamidfaserflor erfolgen.

Die Sensormaterialmischung wird in geeigneter Weise auf den vorbereiteten Bereich des Überzugs aufgetragen. Durch den hier exemplarisch angeführten Tampondruck kann eine homogene Schicht mit einheitlicher Schichtdicke im gewünschten Bereich erzeugt werden. Durch thermische Behandlung kann das Phasenverhalten der flüssigkristallinen Komponenten derart angepasst werden, dass ein hoher Grad an Orientierung in den Ausgangskomponenten des Sensors erhalten wird.

Durch Temperatur und/oder durch chemische Initiierung, jedoch idealerweise durch Licht und damit durch photochemische Vernetzung der reaktiven Komponenten, erfolgt die Stabilisierung der helikalen Überstruktur. Hierfür wird das Gemisch mit Licht einer für den Photoinitiator geeigneten Wellenlängenverteilung bestrahlt, sodass die Radikalbildung umfänglich stattfindet. Hierdurch wird das mechanochrome Sensormaterial erhalten.

Alternativ können elastische photonische Gitter als Sensormaterial eingesetzt werden. Diese lassen sich beispielsweise durch einen Prozess erzeugen, bei dem ein durch Abscheidung von sphärischen Silikapartikeln auf einem Träger gebildete Gitter als Templatphase genutzt werden. Die Dicke der auf diesem Wege erzeugten Schicht ist über die Prozessbedingungen der Abscheidung aus der Silikadispersion gesteuert. Das auf diese Weise erhaltene Hilfsgitter wird getrocknet und in einer Tetramethoxysilanatmosphäre mechanisch stabilisiert. Im Anschluss werden die Hohlräume zwischen den Gitterpunkten mit den monomeren Bestandteilen des elastischen Netzwerkes gefüllt und photochemisch zum elastomeren Netzwerk polymerisiert. Dabei werden bevorzugt im Verhältnis von 10 : 1 : 0,05 Ethylhexylmethacrylat mit dem Vernetzer Ethylenglykoldimethacrylat unter Verwendung des Photoinitiators BAPO gemischt und durch Bestrahlung mit Licht im Bereich von 300 nm zur Reaktion gebracht. Die genutzte monofunktionale Monomereinheit kann durch Lauryl-, Butyl- und Methylderivate, aber auch durch Butyl- und Hexylacrylate, sowie Mischungen dieser Komponenten zur Einstellung der mechanischen Responsivität des Netzwerkes ersetzt werden. Das auf diese Weise erzeugte Komposit bestehend aus Polymernetzwerk und Hilfsgitter kann nun in eine gewünschte Form gebracht werden.

Zur Entfernung der Silika-Templathase dient bevorzugt ein Waschprozeß mit einer 2%-igen wässrigen Fluorwasserstofflösung, welcher das inverse Elastomernetzwerk freigibt. Dieses kann getrocknet weiterverwendet werden und im gewünschten Bereich des Fingersegments integriert werden.

Das Sensormaterial wird bevorzugt durch erneutes Tauchen in die Koagulationshilfsmittellösung und anschließendes Tauchen in die Polymerdispersion vollständig eingeschlossen. Danach wird der Überzug vom Formwerkzeug abgezogen, sodass die zuvor am Formwerkzeug anliegende Polymerschicht nach außen zeigt und die zuletzt erzeugte Polymerschicht dem Nutzer - also dem Finger respektive dessen Hand - zugewandt ist.

## Patentansprüche

1. Fingersegment für einen Handschuh für diagnostische und medizinische Zwecke, enthaltend
ein den Finger des Nutzers umschließendes, elastisches Material und
ein vom elastischen Material eingeschlossenes Sensormaterial,
wobei das Sensormaterial geeignet ist, eine einwirkende mechanische Kraft anzuzeigen und
wobei das Sensormaterial eine für die einwirkende mechanische Kraft charakteristische Änderung erfährt, die für den Nutzer ohne technische Hilfsmittel erkennbar ist,
wobei das Sensormaterial ein Material herstellbar aus einem Gemisch aus einem Flüssigkristall, einer optisch aktiven Substanz, einem Photoinitiator, einem Netzwerkmonomer und einem Vernetzer ist,
**dadurch gekennzeichnet, dass** das Sensormaterial dazu geeignet ist, mechanische Kräfte im Bereich von 1 bis 25 N, die auf das Sensormaterial in unterschiedlichen Auftreffwinkeln wirken, zu visualisieren,
wobei das elastische Material ausgewählt ist aus der Gruppe bestehend aus transluzenten Latexmaterialien, transluzentem Polyethylen, transluzentem Neopren, transluzenten Styren-Butadien-Polymeren und Mischungen hiervon, und
wobei die charakteristische Änderung des Sensormaterials durch die Einwirkung von mechanischer Kraft eine Farbänderung im sichtbaren Bereich des elektromagnetischen Spektrums ist, die nur durch einen zusätzlichen Stimulus ausgelöst wird und die reversibel oder irreversibel erfolgen kann.

2. Fingersegment nach Anspruch 1, **dadurch gekennzeichnet, dass**
das Fingersegment zumindest teilweise und bevorzugt vollständig einen mehrschichtigen Aufbau aufweist, wobei ein dreischichtiger Aufbau besonders bevorzugt ist und insbesondere bevorzugt eine innere Schicht, die das Sensormaterial enthält von zwei äußeren Schichten, die das elastische Material enthalten, eingeschlossen ist;
dabei beträgt die Schichtdicke der Schicht mit dem Sensormaterial bevorzugt von 50 bis 100 µm und besonders bevorzugt von 50 bis 60 µm und die Gesamtdicke des Mehrschichtaufbaus bevorzugt von 150 bis 1000 µm und besonders bevorzugt von 150 bis 500 µm; und/oder
das elastische Material den Finger des Nutzers vollständig umschließt; und/oder
das Fingersegment aus dem elastischen Material und dem Sensormaterial besteht.

3. Fingersegment nach einem der Ansprüche 1 oder 2, **dadurch gekennzeichnet, dass**
das Sensormaterial durch Verfahren ausgewählt aus der Gruppe bestehend aus Rakeln, Tauchen, Sprühen, Drucken und Kleben auf dem elastischen Material als Schicht auf einer Schicht des elastischen Materials fixiert wird, wobei es bei Rakeln, Tauchen, Sprühen und Drucken bevorzugt ist, dass das Sensormaterial vollständig vom elastischen Material umhüllt wird.

4. Fingersegment nach einem der vorhergehenden Ansprüche, **dadurch gekennzeichnet, dass**
das Sensormaterial in allen Bereichen des Fingersegments eine identische Empfindlichkeit aufweist; oder
das Sensormaterial in verschiedenen Bereichen des Fingersegments angebracht ist und jeweils unterschiedliche Empfindlichkeiten aufweist, wobei die Differenz in der Empfindlichkeit mindestens 5 N, bevorzugt mindestens 8 N beträgt,
wobei es bevorzugt ist, dass die Einstellung der Empfindlichkeit des Sensormaterials während des Herstellprozesses erfolgt, besonders bevorzugt durch Materialwahl des Sensormaterials und/oder durch die Einstellung der Schichtdicke.

5. Fingersegment nach einem der vorhergehenden Ansprüche, **dadurch gekennzeichnet, dass**
der zusätzliche Stimulus, der die Farbänderung im sichtbaren Bereich des elektromagnetischen Spektrums auslöst, die Einwirkung von Licht einer Wellenlänger von < 400 nm, bevorzugt < 360 nm, insbesondere im Bereich von 280 bis 400 nm und besonders bevorzugt im Bereich von 280 bis 360 nm, ist.

6. Fingersegment nach einem der vorhergehenden Ansprüche, **dadurch gekennzeichnet, dass**
der Flüssigkristall ausgewählt ist aus der Gruppe bestehend aus Benzoesäurecholesterylester, Cholesterin, Tolan, Alkansäuren, Stilben, Azobenzolen, N-(p-Ethoxy-benzyliden)-p-n-butylanilin, N-(p-Methoxy-benzyliden)-p-n-butylanilin, Derivaten des 4-n-Alkylbenzoesäure-(4-alkyl-phenylesters), 4-Phenylzimtsäure, p-Terphenyl, 1,2-Bis-benzoylethen, Flüssigkristallmischungen von Alkyl-cyanobiphenyl- und Alkoxycyanobiphenylderivaten und Mischungen hiervon; und/oder
die optische aktive Substanz ein 4-(4-Hexyloxybenzoyloxy)benzoat oder ein Cholesterylderivat ist; und/oder
die netzwerkbildenden Monomere Verbindungen aus gewählt aus der Gruppe bestehend aus Benzylmethacrylat, 2-Ethylhexylacrylat, 2-Methoxyethylacrylat, Octadecylacrylat und Reaktivmesogenen und Mischungen hiervon sind; und/oder
der Vernetzer ist ausgewählt ist aus der Gruppe bestehend aus 1,4-Butandiol-diacrylat, Polyethylenglycol-diacrylat, 1,10 Decandioldiacrylat und Mischungen hiervon; und/oder
der Photoinitator ausgewählt ist aus der Gruppe bestehend aus Benzilketalen, α-Dialkoxyacetophenonen, α-Hydroxyalkylphenolen, α-Aminoalkylphenonen, Acylphosphinoxiden, Benzophenonen, Photosäuren und Mischungen hiervon;
wobei es besonders bevorzugt ist, dass ein Gemisch verwendet wird welches den Vernetzer mit bis zu 20 Gew.-%; das netzwerkbildende Monomer und den Flüssigkristall mit bis zu 90 Gew.-%, den Photoinitiator mit bis zu 2 Gew.-% und die optisch aktive Substanz mit bis zu 30 Gew.-% enthält.

7. Fingersegment nach einem der vorhergehenden Ansprüche, **dadurch gekennzeichnet, dass**
die charakteristische Änderung die das Sensormaterial erfährt für Maschinen lesbar und/oder auswertbar ist, bevorzugt ist die charakteristische Änderung hierbei ein Farbwechsel.

8. Fingersegment nach einem der vorhergehenden Ansprüche, **dadurch gekennzeichnet, dass** das Sensormaterial Drücke im Bereich von 0,1 bis 5 bar, bevorzugt 0,5 bis 2,5 bar visualisieren kann, die senkrecht zur Flächennormalen des Sensormaterials oder der das Sensormaterial enthaltenden Schicht wirken.

9. Fingersegment nach einem der vorhergehenden Ansprüche, **dadurch gekennzeichnet, dass**
das Fingersegment geeignet ist
a) anzuzeigen ob eine mechanische Krafteinwirkung erfolgte; und
b) diese Krafteinwirkung zu quantifizieren.

10. Fingersegment nach einem der vorhergehenden Ansprüche, **dadurch gekennzeichnet, dass**
die Intensität der mechanischen Krafteinwirkung durch unterschiedliche Farben visualisiert wird, wobei es bevorzugt ist, dass eine zunehmende Einwirkung der mechanischen Kraft durch eine Blauverschiebung angezeigt wird.

11. Fingersegment nach einem der vorhergehenden Ansprüche, **dadurch gekennzeichnet, dass**
die charakteristische Änderung des Sensormaterials auch in den an die Bereiche in denen mechanische Krafteinwirkung erfolgte direkt benachbarten Bereichen angezeigt wird, bevorzugt durch einen Farbumschlag.

12. Fingersegment nach einem der vorhergehenden Ansprüche, **dadurch gekennzeichnet, dass**
dieses die Erfordernisse der Europäischen Richtlinie 93/42/EWG und/oder der Europäischen Norm EN 455-1 bis 4 erfüllt; und/oder dieses als Fingerling ausgebildet ist.

13. Handschuh der mindestens ein Fingersegment gemäß einem der Ansprüche 1 bis 12 aufweist.

14. Verwendung des Fingersegments nach einem der Ansprüche 1 bis 12 oder des Handschuhs gemäß Anspruch 13 in der Feinmechanik und bei der CD-Herstellung.

## Claims

1. Finger segment for a glove for diagnostic and medical purposes, containing
a resilient material which surrounds the user's finger and
a sensor material which is enclosed by the resilient material,
the sensor material being suitable for indicating an effective mechanical force, and
the sensor material undergoing a change which is characteristic of the effective mechanical force and can be perceived by the user without technical aids,
the sensor material is a material that can be produced from a mixture of a liquid crystal, an optically active substance, a photoinitiator, a network monomer, and a cross-linker,
**characterized in that** the sensor material is suitable for visualizing mechanical forces in the range of from 1 to 25 N which act on the sensor material at different angles of incidence,
the resilient material being selected from the group consisting of translucent latex materials, translucent polyethylene, translucent neoprene, translucent styrene-butadiene polymers, and mixtures thereof, and
the characteristic change in the sensor material due to the effect of mechanical force being a change in color in the visible range of the electromagnetic spectrum which is only triggered by an additional stimulus and is reversible or irreversible.

2. Finger segment according to claim 1, **characterized in that**
the finger segment has a multi-layered structure at least in part and preferably completely, a three-layered structure being particularly preferred and, particularly preferably, an inner layer containing the sensor material being enclosed by two outer layers containing the resilient material;
hereby the layer thickness of the layer containing the sensor material is preferably from 50 to 100 µm and particularly preferably from 50 to 60 µm and the total thickness of the multi-layered structure is preferably from 150 to 1000 µm and particularly preferably from 150 to 500 µm; and/or
the resilient material completely surrounds the user's finger; and/or
the finger segment consists of resilient material and the sensor material.

3. Finger segment according to any one of claims 1 or 2, **characterized in that**
the sensor material is fixed in place on a layer of the resilient material by methods selected from the group consisting of doctoring, dipping, spraying, printing, and bonding to the resilient material as a layer, and during the doctoring, dipping, spraying, and printing, it is preferable for the sensor material to be completely enveloped by the resilient material.

4. Finger segment according to any one of the preceding claims, **characterized in that**
the sensor material has identical sensitivity in all the regions of the finger segment; or
the sensor material is affixed in different regions of the finger segment and has different sensitivities in each of these regions, the difference in the sensitivity being at least 5 N, preferably at least 8 N,
it being preferable for the sensitivity of the sensor material to be set during the production process, particularly preferably by selecting the material for the sensor material and/or by setting the layer thickness.

5. Finger segment according to any one of the preceding claims, **characterized in that**
the additional stimulus triggered by the change in color in the visible range of the electromagnetic spectrum is the effect of light of a wavelength of <400 nm, preferably <360 nm, in particular in the range of 280 to 400 nm and particularly preferably in the range of 280 to 360 nm.

6. Finger segment according to any one of the preceding claims, **characterized in that**
the liquid crystal is selected from the group consisting of benzoic acid cholesteryl esters, cholesterol, tolane, alkane acids, stilbene, azobenzenes, N-(p-ethoxy-benzylidene)-p-n-butylaniline, N-(p-methoxy- benzylidene)-p-n-butylaniline, derivatives of 4-n-alkyl benzoic acid-(4-alkyl-phenylesters), 4-phenyl cinnamic acid, p-terphenyl, 1,2-bis-benzoyl ethene, liquid crystal mixtures of alkyl-cyanobiphenyl and alkoxy-cyanobiphenyl derivatives, and mixtures thereof; and/or
the optical active substance is a 4-(4-hexyloxy benzoyloxy)benzoate or a cholesteryl derivative; and/or
the network-forming monomers are compounds selected from the group consisting of benzyl methacrylate, 2-ethylhexyl acrylate, 2-methoxyethyl acrylate, octadecyl acrylate, and reactive mesogens, and mixtures thereof; and/or
the cross-linker is selected from the group consisting of 1,4-butanediol diacrylate, polyethylene glycol diacrylate, 1,10-decanediol diacrylate, and mixtures thereof; and/or
the photoinitiator is selected from the group consisting of benzilketales, α-dialkoxy acetophenones, α-hydroxyalkyl phenols, α-aminoalkyl phenones, acylphosphine oxides, benzophenones, photoacids, and mixtures thereof;
it being particularly preferable for a mixture to be used which contains the cross-linker at up to 20 wt.%, the network-forming monomer and the liquid crystal at up to 90 wt.%, the photoinitiator at up to 2 wt.%, and the optically active substance at up to 30 wt.%.

7. Finger segment according to any one of the preceding claims, **characterized in that**
the characteristic change that the sensor material undergoes can be read and/or evaluated by machines, and the characteristic change is preferably a change in color in this case.

8. Finger segment according to any one of the preceding claims, **characterized in that** the sensor material can visualize pressures in the range of from 0.1 to 5 bar, preferably 0.5 to 2.5 bar, which take effect perpendicularly to the surface normal of the sensor material or the layer containing the sensor material.

9. Finger segment according to any one of the preceding claims, **characterized in that**
the finger segment is suitable
a) for indicating whether there was a mechanical force effect; and
b) for quantifying this force effect.

10. Finger segment according to any one of the preceding claims, **characterized in that**
the intensity of the mechanical force effect is visualized by different colors, it being preferable for an increasing effect of the mechanical force to be indicated by a blue shift.

11. Finger segment according to any one of the preceding claims, **characterized in that**
the characteristic change in the sensor material is also indicated in the regions directly adjacent to the regions in which there was a mechanical force effect, preferably by a color change.

12. Finger segment according to any one of the preceding claims, **characterized in that**
it meets the requirements of European Directive 93/42/EEC and/or European standard EN 455-1 to 4; and/or it is configured as a finger cot.

13. Glove which comprises at least one finger segment according to any one of claims 1 to 12.

14. Use of the finger segment according to any one of claims 1 to 12 or of the glove according to claim 13 in precision mechanics and CD production.

## Revendications

1. Segment de doigt pour un gant à usage diagnostique et médical, comprenant
un matériau élastique entourant le doigt de l'utilisateur, et
un matériau de détection entouré par le matériau élastique,
le matériau de détection étant adapté pour indiquer une force mécanique appliquée, et
le matériau de détection subissant une modification caractéristique de la force mécanique appliquée, qui est reconnaissable par l'utilisateur sans outil technique,
dans lequel le matériau de détection est un matériau pouvant être fabriqué à partir d'un mélange d'un cristal liquide, d'une substance optiquement active, d'un photo-initiateur, d'un monomère de réseau et d'un agent de réticulation,
**caractérisé en ce que** le matériau de détection est adapté pour visualiser des forces mécaniques dans la plage allant de 1 à 25 N, qui agissent sur le matériau de détection à différents angles d'impact,
dans lequel le matériau élastique est choisi dans le groupe constitué de matériaux en latex translucides, de polyéthylène translucide, de néoprène translucide, de polymères styrène-butadiène translucides et de leurs mélanges, et
la modification caractéristique du matériau de détection sous l'effet d'une force mécanique étant un changement de couleur dans le domaine visible du spectre électromagnétique, qui n'est déclenché que par un stimulus supplémentaire et qui peut être réversible ou irréversible.

2. Segment de doigt selon la revendication 1, **caractérisé en ce que**
le segment de doigt présente au moins partiellement et de préférence entièrement une structure multicouche, une structure à trois couches étant particulièrement préférée et, en particulier, une couche intérieure, qui contient le matériau de détection, étant entourée de deux couches extérieures, qui contiennent le matériau élastique ;
l'épaisseur de la couche contenant le matériau de détection est de préférence de 50 à 100 µm et de manière particulièrement préférée de 50 à 60 µm et l'épaisseur totale de la structure multicouche est de préférence de 150 à 1 000 µm et de manière particulièrement préférée de 150 à 500 µm ; et/ou
le matériau élastique entoure complètement le doigt de l'utilisateur ; et/ou
le segment de doigt est constitué du matériau élastique et du matériau de détection.

3. Procédé selon l'une des revendications 1 ou 2, **caractérisé en ce que**
le matériau de détection est fixé sur le matériau élastique sous forme de couche sur une couche du matériau élastique par des procédés choisis dans le groupe constitué par le raclage, le trempage, la pulvérisation, l'impression et le collage, dans lequel, dans le cas du raclage, du trempage, de la pulvérisation et de l'impression, il est préférable que le matériau de détection soit totalement enveloppé par le matériau élastique.

4. Segment de doigt selon l'une quelconque des revendications précédentes, **caractérisé en ce que**
le matériau de détection présente une sensibilité identique dans toutes les zones du segment de doigt ; ou
le matériau de détection est appliqué dans différentes zones du segment de doigt et présente respectivement des sensibilités différentes, la différence de sensibilité étant d'au moins 5 N, de préférence d'au moins 8 N,
le réglage de la sensibilité du matériau de détection étant de préférence effectué pendant le processus de fabrication, de manière particulièrement préférée par le choix du matériau de détection et/ou par le réglage de l'épaisseur de couche.

5. Segment de doigt selon l'une quelconque des revendications précédentes, **caractérisé en ce que**
le stimulus supplémentaire qui déclenche le changement de couleur dans le domaine visible du spectre électromagnétique est l'action de la lumière d'une longueur d'onde < 400 nm, de préférence < 360 nm, en particulier dans la plage de 280 à 400 nm et de manière particulièrement préférée dans la plage de 280 à 360 nm.

6. Segment de doigt selon l'une quelconque des revendications précédentes, **caractérisé en ce que**
le cristal liquide est choisi dans le groupe constitué par les esters de cholestérol d'acide benzoïque, le cholestérol, le tolane, les acides alcanoïques, le stilbène, les azobenzènes, la N-(p-éthoxy-benzylidène)-p-n-butylaniline, la N-(p-méthoxy-benzylidène)-p-n-butylaniline, les dérivés de l'acide 4-n-alkylbenzoïqueure-(4-alkyl-phénylester), l'acide 4-phénylcinnamique, le p-terphényle, le 1,2-bis-benzoyléthène, les mélanges de cristaux liquides de dérivés d'alkyl-cyanobiphényle et d'alcoxycyanobiphényle et leurs mélanges ; et/ou
la substance optiquement active est un 4-(4-hexyloxybenzoyloxy)benzoate ou un dérivé de cholestéryle ; et/ou
les monomères formant le réseau sont des composés choisis dans le groupe constitué par le méthacrylate de benzyle, l'acrylate de 2-éthylhexyle, l'acrylate de 2-méthoxyéthyle, l'acrylate d'octadécyle et les mésogènes réactifs, et leurs mélanges ; et/ou
l'agent de réticulation est choisi dans le groupe constitué par le diacrylate de 1,4-butanediol, le diacrylate de polyéthylèneglycol, le diacrylate de 1,10-décanediol et leurs mélanges ; et/ou
le photo-initiateur est choisi dans le groupe constitué par les cétals de benzile, les a-dialcoxyacétophénones, les a-hydroxyalkylphénols, les a-aminoalkylphénones, les oxydes d'acylphosphine, les benzophénones, les photoacides et leurs mélanges ;
où il est particulièrement préférable d'utiliser un mélange qui contient l'agent de réticulation jusqu'à 20 % en poids ; le monomère formant le réseau et le cristal liquide jusqu'à 90 % en poids, le photo-initiateur jusqu'à 2 % en poids et la substance optiquement active jusqu'à 30 % en poids.

7. Segment de doigt selon l'une quelconque des revendications précédentes, **caractérisé en ce que**
la modification caractéristique que subit le matériau de détection est lisible et/ou exploitable par des machines, de préférence la modification caractéristique est ici un changement de couleur.

8. Segment de doigt selon l'une des revendications précédentes, **caractérisé en ce que** le matériau de détection peut visualiser des pressions dans la plage allant de 0,1 à 5 bar, de préférence de 0,5 à 2,5 bar, qui agissent perpendiculairement à la normale à la surface du matériau de détection ou de la couche contenant le matériau de détection.

9. Segment de doigt selon l'une quelconque des revendications précédentes, **caractérisé en ce que**
le segment de doigt est adapté pour
a) indiquer si une force mécanique a été exercée ; et
b) quantifier cette force.

10. Segment de doigt selon l'une quelconque des revendications précédentes, **caractérisé en ce que**
l'intensité de la force mécanique est visualisée par différentes couleurs, la préférence étant donnée au fait qu'une action croissante de la force mécanique est indiquée par un décalage vers le bleu.

11. Segment de doigt selon l'une quelconque des revendications précédentes, **caractérisé en ce que**
la modification caractéristique du matériau de détection est également indiquée, de préférence par un changement de couleur, dans les zones directement voisines des zones dans lesquelles s'est produite la force mécanique.

12. Segment de doigt selon l'une quelconque des revendications précédentes, **caractérisé en ce que**
celui-ci répond aux exigences de la directive européenne 93/42/CEE et/ou de la norme européenne EN 455-1 à 4 ; et/ou celui-ci est conçu comme un doigtier.

13. Gant comprenant au moins un segment de doigt selon l'une des revendications 1 à 12.

14. Utilisation du segment de doigt selon l'une des revendications 1 à 12 ou du gant selon la revendication 13 en mécanique de précision et dans la fabrication de CD.
